# EUROPEAN PATENT SPECIFICATION

(11) **EP 2 806 806 B1**
(45) Date of publication and mention of the grant of the patent: **10.04.2019**
(21) Application number: 13702679.5
(22) Date of filing: 14.01.2013
(51) Int. Cl.: A61B 17/16

(54) **MICROFRACTURE PICK**
MIKROFRAKTURIERUNGSPFRIEM
POINTE POUR MICROFRACTURE

(30) Priority: 29.01.2012 US 201261591980 P
(43) Date of publication of application: 03.12.2014
(73) Proprietor: Smith & Nephew, Inc, Memphis, TN 38116 (US)
(72) Inventor: ROGERS, Jon-Paul, North Smithfield, Rhode Island 02896 (US); CALLAHAN, Timothy P., Attleboro, Massachusetts 01073 (US)
(74) Representative: Smith & Nephew
(86) International application number: PCT/US2013/021400
(87) International publication number: WO 2013/112308

(56) References cited:
- WO-A2-2007/106895
- WO-A2-2008/045902
- WO-A2-2009/129272
- US-A1- 2004 073 223

## Description

This patent application claims benefit of the priority of U.S. Provisional Patent Application No. 61/591,980 filed January 29, 2012 entitled MICROFRACTURE PICK.

### FIELD OF THE INVENTION

The subject application relates generally to microfracture stimulation, and more specifically to surgical devices for use in performing microfracture stimulation.

### BACKGROUND

In the human body, articulating joints are surfaced with hyaline cartilage, which is a durable natural material with a low coefficient-of-friction. Such hyaline cartilage surfaces can become damaged over time when subjected to high levels of repeated loading or injury, such as the loading that can occur when a person runs. This is particularly the case for articulating joints in the lower body that are subject to compressive forces, such as the joints located in the ankle, knee, hip, and spine.

In recent years, the resurfacing of cartilage surfaces has been widely studied in the orthopedic industry. One known method of resurfacing cartilage surfaces is referred to as "microfracture stimulation". Instead of replacing damaged hyaline cartilage with an artificial cartilage implant, microfracture stimulation can be performed to stimulate the human body to replace the damaged cartilage with fibrous cartilage tissue (also referred to herein as "fibrocartilage"). Fibrocartilage is generally not as robust as hyaline cartilage, and typically has a higher coefficient-of-friction compared with that of hyaline cartilage. Nonetheless, such fibrocartilage provides many people with reduced pain, enabling them to assume more active lifestyles.

A conventional microfracture pick for use in performing microfracture stimulation has a handle, a shaft coupled to the handle, and a sharp, optionally angled tip disposed at a distal end of the shaft. For example, conventional microfracture picks can have tips that are optionally bent at angles of about 20°, 40°, 60°, or 90° relative to the longitudinal axis of the shaft. In a typical mode of operation, microfracture stimulation first involves the removal of the damaged layer of cartilage. The thickness of the damaged cartilage layer can typically vary from about 1 mm to 6 mm. The sharp tip of the microfracture pick is then driven about 2 mm to 5 mm through underlying subchondral bone in the region of the removed layer of cartilage to reach a blood supply. The microfracture pick is then removed, causing a small channel to remain in the subchondral bone. The microfracture pick is typically used to create a series of such channels through the subchondral bone. As a result, blood eventually travels along the series of channels and clots in the region of the removed cartilage layer, ultimately causing the formation of fibrocartilage. WO2007106895 discloses a microfracture pick having a shaft and a handle. The shaft has a proximal end and a distal end, wherein the distal end includes an angled tip. The handle has a body with a first end portion, a second end portion, and an impact surface located therebetween.

When a surgeon uses such a conventional microfracture pick to perforate the subchondral bone of a patient, he or she may experience difficulties manually advancing the sharp tip through a hard cortical layer of the bone. This can be problematic since not advancing the microfracture pick deep enough into the bone may prohibit the formation of fibrocartilage in the region of the removed cartilage layer. To aid in advancing the sharp tip of the microfracture pick through the subchondral bone, surgeons have traditionally used a hammer or mallet to strike an end of the handle of the microfracture pick, while applying a downward pressure to the handle. However, such use of a hammer or mallet has drawbacks in that it can produce a shear force at the tip of the microfracture pick, potentially causing the tip to become broken or otherwise damaged. Such a broken tip can become a loose body in the surgical site, and can cause a delay in the progress of the microfracture procedure. In addition, the tip can skive across the bone surface, potentially causing the microfracture pick to impinge on and possibly damage surrounding tissue surfaces.

To address this problem, a strike plate can be directly attached to the shaft or handle of the conventional microfracture pick. Such a strike plate is typically attached to the shaft or handle perpendicular to the direction of the sharp tip of the microfracture pick. When a surgeon strikes the strike plate with a hammer or mallet, the resulting force causes the tip of the microfracture pick to advance through the subchondral bone. Directly attaching a strike plate to the shaft or handle of a microfracture pick also has drawbacks, however, in that it can make the microfracture pick heavy and cumbersome. The act of striking the strike plate so close to the patient's body can also be problematic, especially when performed near delicate joint access locations such as the ankle. The strike plate can also interfere with the microfracture procedure by preventing complete access to the surgical site, potentially making it extremely difficult for the surgeon to treat the entire affected surface area of the bone.

It would therefore be desirable to have a microfracture pick that avoids at least some of the drawbacks of the conventional microfracture picks discussed above.

### SUMMARY

In accordance with the subject application, a surgical device (referred to herein as a "microfracture pick") is disclosed that has features configured to aid a user in advancing the microfracture pick through bone. The invention is defined by the appended claims.

In one aspect, the disclosed microfracture pick includes at least one elongated member such as a shaft having a proximal end and a distal end, a sharp, optionally angled tip disposed at the distal end of the shaft, an optional handle coupled to the proximal end of the shaft, and at least one engaging feature disposed at one or more locations on the shaft or handle for engaging a complementary feature of a strike instrument. In an exemplary aspect, the strike instrument includes at least one elongated member such as a shaft having a proximal end and a distal end, the complementary feature disposed at the distal end of the shaft, an optional handle having a proximal end as well as a distal end coupled to the proximal end of the shaft, and an impact surface disposed at the proximal end of the shaft or handle. In a further exemplary aspect, the engaging feature disposed on the shaft or handle of the microfracture pick is configured as a receptacle, and the complementary feature of the strike instrument is configured to operatively engage the receptacle on the microfracture pick. In another exemplary aspect, the complementary feature of the strike instrument is configured as a receptacle, and the engaging feature disposed on the shaft or handle of the microfracture pick is configured to operatively engage the receptacle on the strike instrument.

In another aspect, a system for use in performing a microfracture procedure is disclosed that includes a strike instrument having an elongated member such as a handle with a proximal end, a distal end, and a hole located near the distal end of the handle, a strike pin configured to pass through the hole in the handle, an impact surface located at one end of the strike pin, and a complementary feature located at the other end of the strike pin. The system further includes a surgical device having an optional handle, an elongated member such as a shaft with a distal end, an engaging feature located on the shaft or handle, and a sharp, optionally angled tip located at the distal end of the shaft. The engaging feature of the surgical device is operative to engage the complementary feature of the strike instrument. Moreover, the handle of the strike instrument is configured to be disposed generally parallel to the longitudinal axis of the surgical device to facilitate engagement of the engaging feature with the complementary feature. Upon striking the impact surface of the strike instrument in a direction generally parallel to the direction of the tip of the surgical device, a force is produced that is translated via the shaft of the surgical device through the tip.

Also disclosed, but not part of the invention, is a method of performing a microfracture procedure that includes providing a surgical device having at least one elongated member such as a shaft with a proximal end and a distal end, a sharp, optionally angled tip disposed at the distal end of the shaft, an optional handle coupled to the proximal end of the shaft, and at least one engaging feature disposed at one or more locations on the shaft or handle for engaging a complementary feature of a strike instrument. The method also includes locating the tip at a desired point of stimulation, and striking an impact surface of the strike instrument in a direction generally parallel to the direction of the tip to produce a force that is translated via the shaft through the tip. The tip is then removed from the desired point of stimulation. Such use of a strike instrument in performing a microfracture procedure allows a surgeon to place the microfracture perforation at the desired point of stimulation with increased accuracy. Such use of the strike instrument also reduces the amount of force that the surgeon must manually apply to the microfracture pick to achieve the desired microfracture perforation.

By providing a microfracture pick having an elongated member with a proximal end, a distal end, a sharp, optionally angled tip disposed at the distal end of the elongated member, and at least one engaging feature disposed at one or more locations on the elongated member for engaging a complementary feature of a strike instrument, a user can use the strike instrument to produce a force that is translated via the elongated member of the microfracture pick through the tip, thereby making penetration of the tip through bone more effective. As a result, the number of instances of fracturing the tip during use can be reduced. Further, the number of instances of skiving the tip along the bone surface during use can be substantially eliminated.

Other features, functions, and aspects of the invention will be evident from the Detailed Description that follows.

### BRIEF DESCRIPTION OF THE DRAWINGS

The accompanying drawings, which are incorporated in and constitute a part of this specification, illustrate one or more embodiments described herein and, together with the Detailed Description, explain these embodiments. In the drawings:
FIG. 1 is a side view illustrating an exemplary microfracture pick, and an exemplary strike instrument configured to operatively engage the microfracture pick, in accordance with the subject application;
FIG. 2 is a perspective view illustrating the microfracture pick of FIG. 1;
FIG. 3 is a perspective view illustrating the microfracture pick of FIG. 1, and the strike instrument of FIG. 1;
FIG. 4 is a sectional view illustrating the microfracture pick of FIG. 1, and the strike instrument of FIG. 1, including an engaging feature of the microfracture pick, and a complementary feature of the strike instrument;
FIG. 5 is a perspective view illustrating a first alternative embodiment of the microfracture pick of FIG. 1;
FIG. 6 is a perspective view illustrating a second alternative embodiment of the microfracture pick of FIG. 1;
FIG. 7 is a perspective view illustrating an exemplary microfracture pick, and an alternative embodiment of the strike instrument of FIG. 1;
FIG. 8 is another perspective view illustrating the microfracture pick of FIG. 7, and the strike instrument of FIG. 7; and
FIG. 9 is a flow diagram illustrating a method of performing a microfracture procedure using the microfracture pick of FIG. 1.

### DETAILED DESCRIPTION

A microfracture pick is disclosed having features configured to aid a user in advancing the microfracture pick through bone. The microfracture pick has a shaft with a proximal end, a distal end, a sharp, optionally angled tip located at the distal end of the shaft, and at least one engaging feature disposed at one or more locations on the shaft for engaging a complementary feature of a strike instrument. By striking an impact surface of the strike instrument, the user can produce a force that is translated via the shaft of the microfracture pick through its tip, thereby making penetration of the tip through the bone more effective.

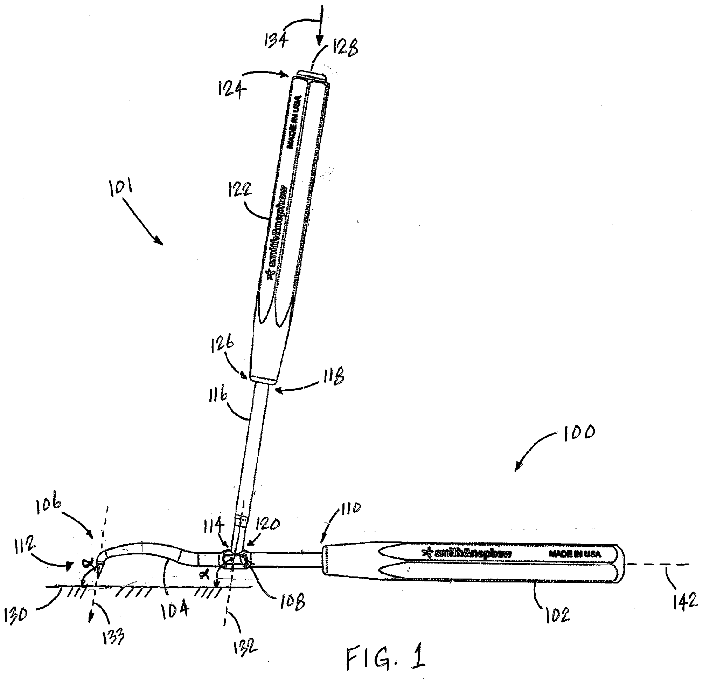

FIG. 1 depicts an illustrative embodiment of a microfracture pick 100, in accordance with the subject application. As shown in FIG. 1, the microfracture pick 100 has a proximal portion that includes an elongated handle 102, and a distal portion that includes a generally elongated shaft 104, which has a proximal end 110 and a distal end 112. The handle 102 is coupled to the proximal end 110 of the shaft 104. The microfracture pick 100 further includes a sharp, optionally angled tip 106 located at the distal end 112 of the shaft 104, and an engaging feature 108 disposed at a fixed location on the shaft 104 for engaging a complementary feature 114 of a strike instrument 101. The strike instrument 101 can include a shaft 116 that has a proximal end 118 and a distal end 120, and the complementary feature 114 can be located at the distal end 120 of the shaft 116. The strike instrument 101 can further include a handle 122 having a proximal end 124 and a distal end 126 coupled to the proximal end 118 of the shaft 116, and an impact surface 128 located at the proximal end 124 of the handle 122.

It is noted that the handle 122, the shaft 116, and the impact surface 128 of the strike instrument 101 can be implemented as a single component. Likewise, the handle 102 and the shaft 104 of the microfracture pick 100 can be implemented as a single component. It is also noted that the shaft 104 of the microfracture pick 100, as well as the shaft 116 of the strike instrument 101, can be made from machined medical grade material such as hardened stainless steel, or any other suitable material. Further, the handle 102 of the microfracture pick 100, as well as the handle 122 of the strike instrument 101, can have a cylindrical shape, or any other suitable shape. In some embodiments, the handle 102 of the microfracture pick 100 may be omitted. Moreover, the tip 106 can be optionally bent at an angle of about 20°, 40°, 60°, 90°, or any other suitable angle, relative to the longitudinal axis 142 of the microfracture pick 100. For example, the tip 106 can be optionally bent at an angle greater than 90° relative to the longitudinal axis 142 by bending the tip 106 back toward the handle 102.

During use, the direction 133 of the sharp tip 106 of the microfracture pick 100 can be aligned at an angle α relative to a surface 130 (see FIG. 1), which can correspond to the surface of bone. Once the complementary feature 114 of the strike instrument 101 is engaged with the engaging feature 108 of the microfracture pick 100, the longitudinal axis 132 the strike instrument 101 can be aligned at about the same angle α relative to the surface 130. Accordingly, when a user (*e.g.,* a surgeon) strikes the impact surface 128 of the strike instrument 101 with a hammer, mallet, or any other suitable striking implement, in a direction substantially parallel to the direction 133 of the tip 106, a force 134 is produced that is translated via the shaft 104 of the microfracture pick 100 through the tip 106.

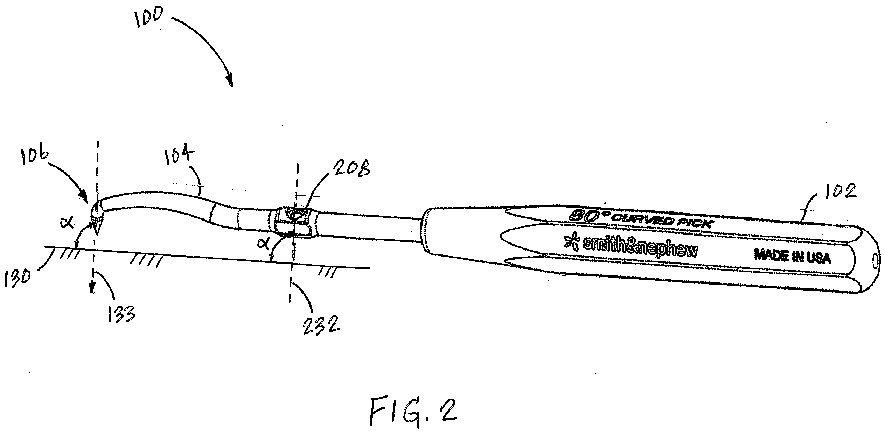

FIG. 2 depicts a perspective view of the microfracture pick 100, including the handle 102, the shaft 104, the sharp, optionally angled tip 106, and the engaging feature (see reference numeral 108; FIG. 1), which is configured as a receptacle 208. In this illustrative embodiment, the complementary feature 114 of the strike instrument 101 (see FIG. 1) is configured to operatively engage the receptacle 208 of the microfracture pick 100. To assure that the strike instrument 101 is aligned at about the same angle α as the tip 106 relative to the surface 130 (see FIG. 1) while the complementary feature 114 is engaged with the receptacle 208, the receptacle 208 can be configured such that its axis 232 is substantially parallel to the direction 133 of the tip 106. Accordingly, if the surgeon experiences difficulties advancing the tip 106 of microfracture pick 100 through bone, he or she can insert the complementary feature 114 of the strike instrument 101 into the receptacle 208 of the microfracture pick 100, and strike the impact surface 128 of the strike instrument 101 one or more times with a hammer or mallet to produce a force along the axis 232 of the receptacle 208, thereby facilitating advancement of the tip 106 through the bone.

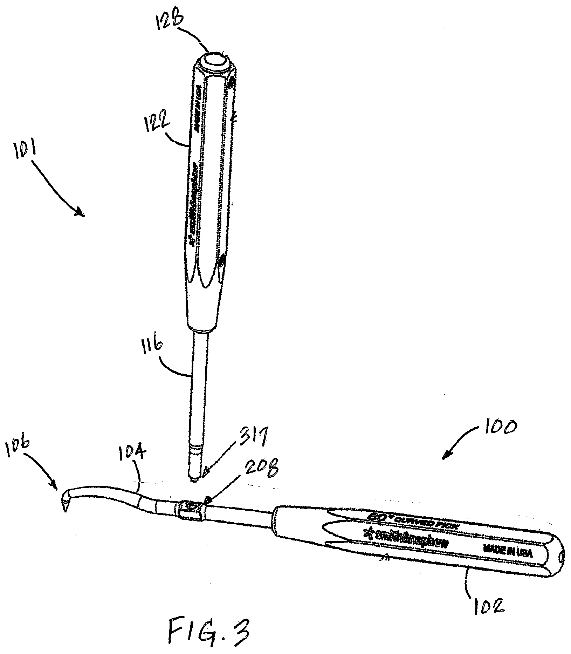

FIG. 3 depicts a perspective view illustrating the microfracture pick 100 and the strike instrument 101, in which the complementary feature (see reference numeral 114; FIG. 1) is configured to include an optional nub 317 that can be temporarily or permanently fixated to the receptacle 208 of the microfracture pick 100. For example, the optional nub 317, or an end of the shaft 116 without the nub 317, can be temporarily or permanently fixated to the receptacle 208 by way of threading, a press fit, a lever lock, a cam lock, a snap fit, a set screw, a taper fit, a luer lock (with or without taper), or any other suitable mechanism.

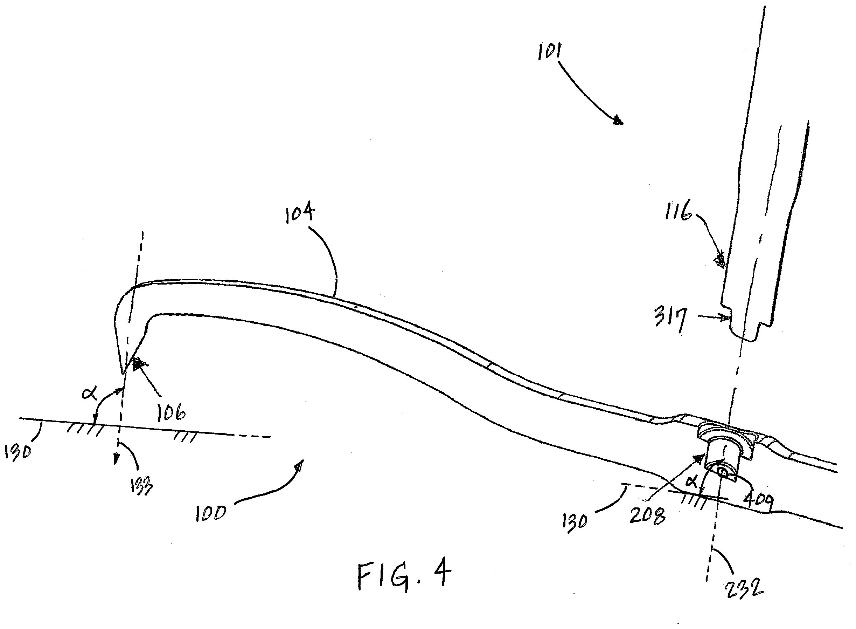

FIG. 4 depicts a sectional view illustrating the microfracture pick 100 including the receptacle 208, as well as the strike instrument 101 including the optional nub 317. In the sectional view of FIG. 4, the receptacle 208 is illustrated as being substantially internal to the shaft 104 of the microfracture pick 100. To assure that the strike instrument 101 is aligned at about the same angle α as the sharp, optionally angled tip 106 relative to the surface 130 while the nub 317 is engaged with the receptacle 208, the receptacle 208 can be configured such that its axis 232 is substantially parallel to the direction 133 of the tip 106. For example, the nub 317 can be configured as a male connector or any other suitable connector, and the receptacle 208 can be configured as a female connector or any other suitable connector. In some embodiments, the microfracture pick 100 can be configured to include a male connector, and the strike instrument 101 can be configured to include a female connector for engaging the male connector of the microfracture pick 100. Such male and female connectors can be temporarily or permanently fixated to one another by way of threading, a press fit, a lever lock, a cam lock, a snap fit, a set screw, a taper fit, a luer lock (with or without taper), or any other suitable mechanism. Further, the receptacle 208 can be configured to include an external drain hole 409 for sterilization and/or dry time purposes, as well as for avoiding a possible build-up of fluid in the receptacle 208 during use. For example, the external drain hole 409 can have a diameter of about 2 mm, or any other suitable diameter.

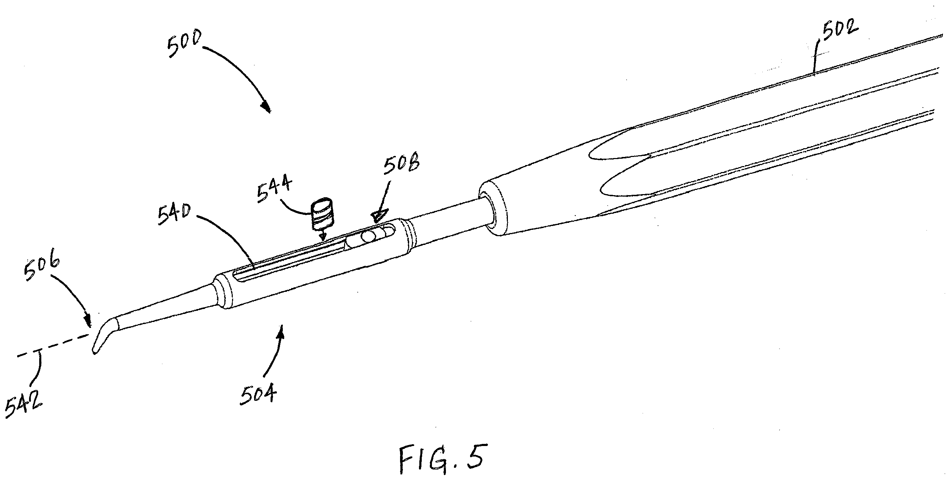

Having described the above illustrative embodiments of the disclosed microfracture pick, other alternative embodiments or variations may be made. For example, it was described with reference to FIG. 1 that the microfracture pick 100 includes the engaging feature 108 disposed at a fixed location on the shaft 104 for engaging the complementary feature 114 of the strike instrument 101. FIG. 5 depicts an alternative embodiment of a microfracture pick 500 that includes a handle 502, a shaft 504, a sharp, optionally angled tip 506, and an engaging feature 508. As shown in FIG. 5, the shaft 504 is configured to incorporate a channel 540 along at least a portion of its length, and the engaging feature 508 is configured to engage and slide along the channel 540 to a desired location on the shaft 504. The channel 540 is substantially parallel to the longitudinal axis 542 of the shaft 504. Further, the engaging feature 508 can be configured to include a receptacle like the receptacle 208 of FIG. 2. Once the engaging feature 508 is slid or otherwise moved along the channel 540 to the desired location on the shaft 504, the engaging feature 508 can be temporarily or permanently fixated at that location on the shaft 504 by at least one stop member 544, which, as shown in FIG. 5, can be inserted into or otherwise engaged with the channel 540 adjacent the engaging feature 508, or by any other suitable mechanism. In alternative embodiments, the engaging feature 508 can be temporarily or permanently fixated at a desired location on the shaft 504. In further alternative embodiments, the engaging feature 508 can be removed if it is not required to perform the microfracture procedure.

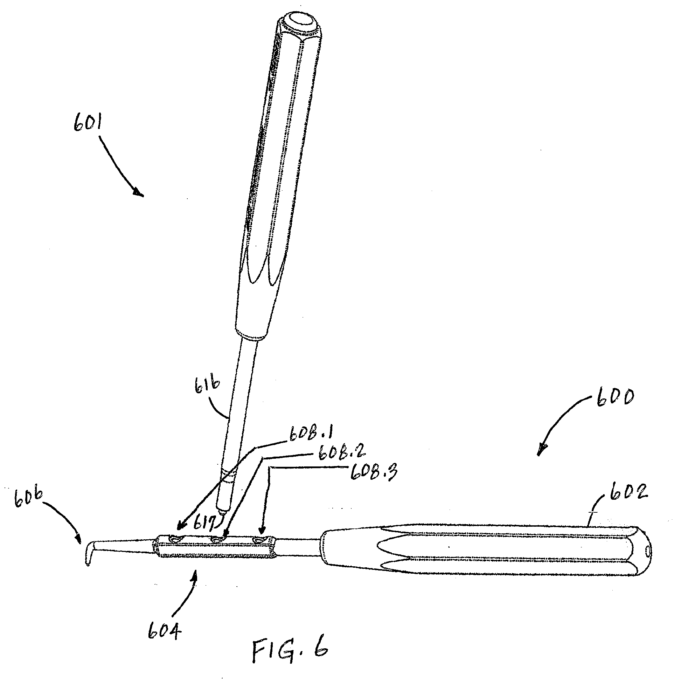

FIG. 6 depicts another alternative embodiment of a microfracture pick 600 that includes a handle 602, a shaft 604, a sharp, optionally angled tip 606, and a plurality of engaging features 608.1, 608.2, 608.3 disposed at a plurality of fixed locations, respectively, on the shaft 604. FIG. 6 depicts three (3) such engaging features on the shaft 604 for purposes of illustration. It should be understood, however, that the microfracture pick 600 can include any suitable number of engaging features disposed at respective locations on the shaft 604. For example, each of the plurality of engaging features 608.1, 608.2, 608.3 can be configured to include a receptacle like the receptacle 208 of FIG. 2. FIG. 6 further depicts a strike instrument 601, in which the complementary feature is configured to include an optional nub 617 at an end of a shaft 616 that can be temporarily or permanently fixated to a selected one of the plurality of engaging features 608.1, 608.2, 608.3 on the shaft 604. For example, the optional nub 617, or the end of the shaft 616 without the nub 617, can be temporarily or permanently fixated to the selected engaging feature 608.1, 608.2, or 608.3 by way of threading, a press fit, a lever lock, a cam lock, a snap fit, a set screw, a taper fit, a luer lock (with or without taper), or any other suitable mechanism.

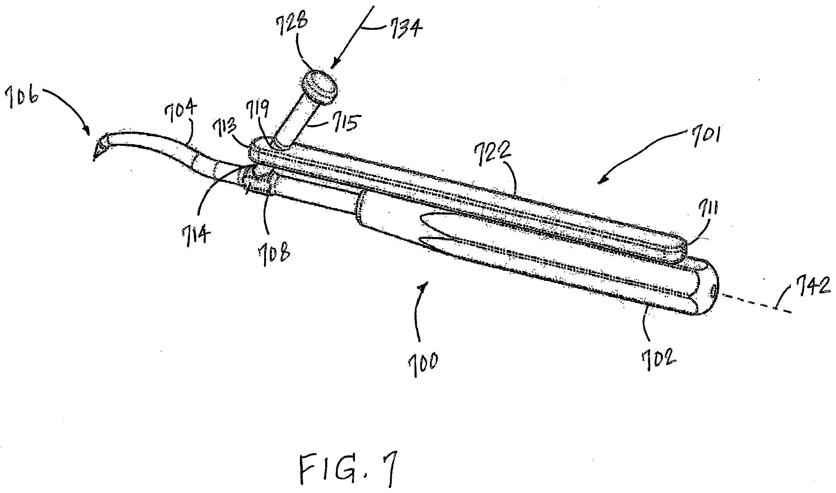

FIG. 7 depicts an alternative embodiment of a strike instrument 701, which includes a complementary feature 714 for engaging an engaging feature 708 of a microfracture pick 700. As shown in FIG. 7, the strike instrument 701 includes a handle 722 having a proximal end 711, a distal end 713, and a hole 719 located near the distal end 713 of the handle 722. The strike instrument 701 further includes a strike pin 715 configured to pass snugly through the hole 719, the complementary feature 714 located at one end of the strike pin 715, and an impact surface 728 located at the other end of the strike pin 715. It is noted that the handle 722, the strike pin 715, and the impact surface 728 of the strike instrument 701 can be implemented as a single component. As further shown in FIG. 7, the microfracture pick 700 includes a handle 702, a shaft 704, the engaging feature 708 located on the shaft 704, and a sharp, optionally angled tip 706 located at a distal end of the shaft 704. It is noted that the strike pin 715 can be temporarily or permanently fixated in the hole 719 through the handle 722 of the strike instrument 701.

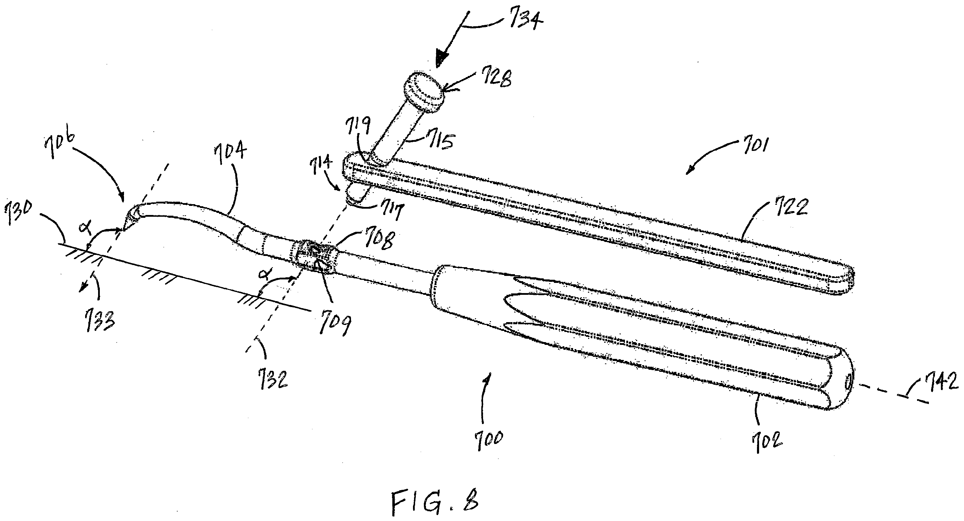

FIG. 8 depicts the microfracture pick 700 and the strike instrument 701, in which the complementary feature 714 of the strike instrument 701 is configured to include an optional nub 717, and the engaging feature 708 of the microfracture pick 700 is configured to include a receptacle 709. For example, the optional nub 717, or an end of the strike pin 715 without the nub 717, can be temporarily or permanently fixated to the receptacle 709 of the microfracture pick 700 by way of threading, a press fit, a lever lock, a cam lock, a snap fit, a set screw, a taper fit, a luer lock (with or without taper), or any other suitable mechanism.

During use, the handle 722 of the strike instrument 701 can be temporarily or permanently fixated to the handle 702 of the microfracture pick 700 substantially parallel to its longitudinal axis 742, thereby allowing the surgeon to hold both the handle 722 of the strike instrument 701 and the handle 702 of the microfracture pick 700 with the same hand. For example, the handle 722 of the strike instrument 701 can be temporarily or permanently fixated to the handle 702 of the microfracture pick 700 by way of threading, a press fit, a lever lock, a cam lock, a snap fit, a set screw, a taper fit, a luer lock (with or without taper), a bayonet mount, or any other suitable mechanism. Further, the strike pin 715 can pass through the hole 719 in the handle 722 to engage the nub 717 of the strike instrument 701 with the receptacle 709 of the microfracture pick 700. To assure that the strike pin 715 is aligned at about the same angle α as the sharp tip 706 relative to a surface 730 while the nub 717 is engaged with the receptacle 709, the receptacle 709 can be configured such that its axis 732 is substantially parallel to the direction 733 of the tip 706. Accordingly, if the surgeon experiences difficulties advancing the tip 706 of microfracture pick 700 through bone, he or she can strike the impact surface 728 of the strike pin 715 one or more times with a hammer or mallet to produce a force 734 along the axis 732 of the receptacle 709, thereby facilitating advancement of the tip 706 through the bone.

It was further described herein that the microfracture pick could include a receptacle on its shaft, and the strike instrument could include an optional nub on its shaft for engaging the receptacle. In further alternative embodiments, the microfracture pick can include at least one engaging feature configured like a nub at a fixed or movable location on its shaft, and the strike instrument can include at least one complementary feature configured like a receptacle on its shaft. For example, such a receptacle on the shaft of the strike instrument can have a forked configuration for cradling the nub on the shaft of the microfracture pick. Moreover, the microfracture pick can alternatively include a nub or receptacle, such as a male or female connector, on its handle for engaging a complementary feature on the strike instrument. Likewise, the strike instrument can alternatively include a nub or receptacle, such as a male or female connector, on its handle for complementarily engaging an engaging feature on the microfracture pick.

It was also described herein that the microfracture pick could include a plurality of engaging features disposed at respective locations on its shaft for engaging a complementary feature of the strike instrument. In further alternative embodiments, the plurality of engaging features of the microfracture pick can be configured as respective receptacles having parallel or non-parallel axes. In addition, the plurality of engaging features of the microfracture pick can each be configured to accept a different configuration of the complementary feature of the strike instrument.

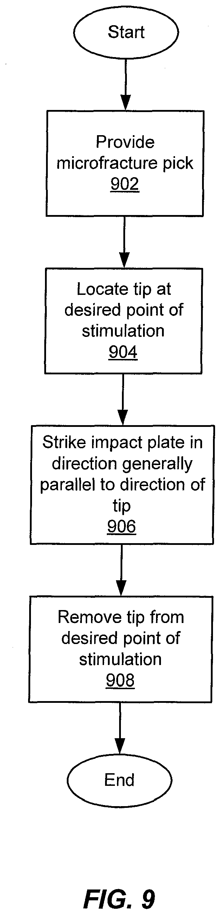

Disclosed but not forming part of the invention is a method of performing a microfracture procedure, using the disclosed microfracture pick, as described below with reference to FIG. 9. As depicted in block 902, the method includes providing a microfracture pick having a shaft with a proximal end and a distal end, an optionally angled tip disposed at the distal end of the shaft, a handle coupled to the proximal end of the shaft, and at least one engaging feature disposed at one or more locations on the shaft for engaging a complementary feature of a strike instrument. As depicted in block 904, the tip is located at a desired point of stimulation. For example, while locating the tip at the desired point of stimulation, at least a portion of the shaft may be inserted into an arthroscopic cannula. As depicted in block 906, an impact surface of the strike instrument is struck by a hammer or mallet in a direction generally parallel to the direction of the tip to produce a force that is translated via the shaft through the tip at the desired point of stimulation. As depicted in block 908, the tip is then removed from the desired point of stimulation.

Although the above illustrative embodiments of the disclosed microfracture pick have been described for use in the context of resurfacing cartilage surfaces, they can also be used for perforating the subchondral bone in the subtalar/talus space (ankle) in arthrodesis procedures. In such arthrodesis procedures, a surgeon typically removes the cartilage on both the subtalar bone and the talus bone, and uses the microfracture pick to perforate the subtalar and talus bones in multiple places to promote bleeding. A screw is then delivered between the subtalar and talus bones to fuse the two bones together.

## Claims

1. A surgical device (100, 500, 600, 700) for use in performing a microfracture procedure, comprising:
an elongated member (104, 504, 604, 704) having a proximal end (110) and a distal end (112);
an angled tip (106, 506, 606, 706) disposed adjacent the distal end of the elongated member; and
at least one engaging feature (108, 508, 608.1, 608.2, 608.3, 708) disposed at one or more locations on the elongated member for engaging a complementary feature (114, 714) of a strike instrument (101, 701),
whereby, upon striking an impact surface (728) of the strike instrument in a direction generally parallel to a direction of the tip, a force is produced that is translated via the elongated member through the tip: and
wherein the engaging feature is configured as one of a female connector and a male connector.

2. The surgical device of claim 1 wherein the engaging feature is configured as a receptacle (208).

3. The surgical device of claim 2 wherein the receptacle has an axis that is generally parallel to the direction of the tip.

4. The surgical device of claim 2 or claim 3 wherein the receptacle has an external drain hole (409).

5. The surgical device of claim 1 wherein the elongated member includes a channel (540) along at least a portion of its length, and the engaging feature is configured to engage and slide along the channel to a desired location on the elongated member.

6. The surgical device of claim 5 further comprising:
at least one stop member operative to fixate the engaging feature at the desired location on the elongated member.

7. The surgical device of claim 1 further comprising:
a plurality of engaging features disposed at respective locations on the elongated member, each engaging feature for engaging the complementary feature of the strike instrument.

8. The surgical device of claim 7 wherein at least some of the engaging features have parallel axes.

9. The surgical device of claim 7 wherein at least some of the engaging features have non-parallel axes.

10. The surgical device of claim 7 wherein at least some of the engaging features are configured to engage different configurations of the complementary feature of the strike instrument.

11. A system for use in performing a microfracture procedure, comprising:
a strike instrument (701) including an elongated member having a proximal end, a distal end, and a hole (719) in the elongated member near its distal end, a strike pin (715) configured to pass through the hole in the elongated member, and an impact surface located at one end of the strike pin; and
a surgical device (700) according to any preceding claim,
wherein the strike instrument further includes a complementary feature located at another end of the strike pin,
wherein the engaging feature of the surgical device is operative to engage the complementary feature of the strike instrument, and
wherein the elongated member of the strike instrument is configured to be disposed generally parallel to a longitudinal axis of the surgical device to allow engagement of the engaging feature with the complementary feature,
whereby, upon striking the impact surface of the strike instrument in a direction generally parallel to a direction of the tip of the surgical device, a force is produced that is translated via the elongated member of the surgical device through the tip.

12. The system of claim 11 wherein the elongated member of the strike instrument is configured to be at least temporarily fixated to the surgical device.

13. The system of claim 11 wherein the engaging feature of the surgical device is configured as a female connector, and the complementary feature of the strike instrument is configured as a male connector.

14. The system of claim 11 wherein the engaging feature of the surgical device is configured as a male connector, and the complementary feature of the strike instrument is configured as a female connector.

## Patentansprüche

1. Eine chirurgische Vorrichtung (100, 500, 600, 700) zur Verwendung beim Durchführen eines Mikrofrakturverfahrens, die Folgendes beinhaltet:
ein längliches Element (104, 504, 604, 704), das ein proximales Ende (110) und ein distales Ende (112) aufweist;
eine abgewinkelte Spitze (106, 506, 606, 706), die neben dem distalen Ende des länglichen Elements angeordnet ist; und
mindestens ein Eingriffsmerkmal (108, 508, 608.1, 608.2, 608.3, 708), das an einer oder mehreren Stellen auf dem länglichen Element angeordnet ist, zum Eingriff mit einem komplementären Merkmal (114, 714) eines Schlaginstruments (101, 701), wobei beim Schlagen auf eine Auftrefffläche (728) des Schlaginstruments in einer zu einer Richtung der Spitze im Allgemeinen parallelen Richtung eine Kraft erzeugt wird, die über das längliche Element durch die Spitze übertragen wird; und
wobei das Eingriffsmerkmal entweder als ein aufnehmendes Verbindungsstück oder ein einzusteckendes Verbindungsstück konfiguriert ist.

2. Chirurgische Vorrichtung gemäß Anspruch 1, wobei das Eingriffsmerkmal als eine Aufnahme (208) konfiguriert ist.

3. Chirurgische Vorrichtung gemäß Anspruch 2, wobei die Aufnahme eine Achse aufweist, die zu der Richtung der Spitze im Allgemeinen parallel verläuft.

4. Chirurgische Vorrichtung gemäß Anspruch 2 oder Anspruch 3, wobei die Aufnahme ein externes Abflussloch (409) aufweist.

5. Chirurgische Vorrichtung gemäß Anspruch 1, wobei das längliche Element einen Kanal (540) entlang mindestens einem Abschnitt seiner Länge umfasst und das Eingriffsmerkmal zum Eingriff mit dem Kanal und zum Gleiten entlang dem Kanal zu einer gewünschten Stelle auf dem länglichen Element konfiguriert ist.

6. Chirurgische Vorrichtung gemäß Anspruch 5, die ferner Folgendes beinhaltet:
mindestens ein Stoppelement, das betriebsfähig ist, um das Eingriffsmerkmal an der gewünschten Stelle auf dem länglichen Element zu fixieren.

7. Chirurgische Vorrichtung gemäß Anspruch 1, die ferner Folgendes beinhaltet:
eine Vielzahl von Eingriffsmerkmalen, die an entsprechenden Stellen auf dem länglichen Element angeordnet sind, wobei jedes Eingriffsmerkmal zum Eingriff mit dem komplementären Merkmal des Schlaginstruments dient.

8. Chirurgische Vorrichtung gemäß Anspruch 7, wobei mindestens einige der Eingriffsmerkmale parallele Achsen aufweisen.

9. Chirurgische Vorrichtung gemäß Anspruch 7, wobei mindestens einige der Eingriffsmerkmale nicht-parallele Achsen aufweisen.

10. Chirurgische Vorrichtung gemäß Anspruch 7, wobei mindestens einige der Eingriffsmerkmale zum Eingriff mit unterschiedlichen Konfigurationen des komplementären Merkmals des Schlaginstruments konfiguriert sind.

11. Ein System zur Verwendung beim Durchführen eines Mikrofrakturverfahrens, das Folgendes beinhaltet:
ein Schlaginstrument (701), umfassend ein längliches Element, das ein proximales Ende, ein distales Ende und ein Loch (719) in dem länglichen Element nahe seinem distalen Ende aufweist, einen Schlagstift (715), der konfiguriert ist, um durch das Loch in dem länglichen Element durchzugehen, und eine Auftrefffläche, die sich an einem Ende des Schlagstifts befindet; und
eine chirurgische Vorrichtung (700) gemäß einem der vorhergehenden Ansprüche,
wobei das Schlaginstrument ferner ein komplementäres Merkmal umfasst, das sich an einem anderen Ende des Schlagstifts befindet,
wobei das Eingriffsmerkmal der chirurgischen Vorrichtung zum Eingriff mit dem komplementären Merkmal des Schlaginstruments betriebsfähig ist und
wobei das längliche Element des Schlaginstruments konfiguriert ist, um im Allgemeinen parallel zu einer Längsachse der chirurgischen Vorrichtung angeordnet zu sein, um den Eingriff des Eingriffsmerkmals mit dem komplementären Merkmal zu ermöglichen,
wobei beim Schlagen auf die Auftrefffläche des Schlaginstruments in einer zu einer Richtung der Spitze der chirurgischen Vorrichtung im Allgemeinen parallelen Richtung eine Kraft erzeugt wird, die über das längliche Element der chirurgischen Vorrichtung durch die Spitze übertragen wird.

12. System gemäß Anspruch 11, wobei das längliche Element des Schlaginstruments konfiguriert ist, um mindestens zeitweise an der chirurgischen Vorrichtung fixiert zu sein.

13. System gemäß Anspruch 11, wobei das Eingriffsmerkmal der chirurgischen Vorrichtung als ein aufnehmendes Verbindungsstück konfiguriert ist und das komplementäre Merkmal des Schlaginstruments als ein einzusteckendes Verbindungsstück konfiguriert ist.

14. System gemäß Anspruch 11, wobei das Eingriffsmerkmal der chirurgischen Vorrichtung als ein einzusteckendes Verbindungsstück konfiguriert ist und das komplementäre Merkmal des Schlaginstruments als ein aufnehmendes Verbindungsstück konfiguriert ist.

## Revendications

1. Un dispositif chirurgical (100, 500, 600, 700) pour une utilisation dans la réalisation d'une procédure de microfracture, comprenant :
un élément allongé (104, 504, 604, 704) ayant une extrémité proximale (110) et une extrémité distale (112) ;
une pointe angulaire (106, 506, 606, 706) disposée de manière adjacente à l'extrémité distale de l'élément allongé ; et
au moins un attribut de mise en prise (108, 508, 608.1, 608.2, 608.3, 708) disposé au niveau d'un ou de plusieurs emplacements sur l'élément allongé pour se mettre en prise avec un attribut complémentaire (114, 714) d'un instrument de frappe (101, 701), moyennant quoi, au moment où l'on frappe une surface d'impact (728) de l'instrument de frappe dans une direction généralement parallèle à une direction de la pointe, une force est produite qui est translatée via l'élément allongé à travers la pointe ; et
dans lequel l'attribut de mise en prise est configuré sous la forme d'un attribut parmi un raccord femelle et un raccord mâle.

2. Le dispositif chirurgical de la revendication 1 dans lequel l'attribut de mise en prise est configuré sous la forme d'un réceptacle (208).

3. Le dispositif chirurgical de la revendication 2 dans lequel le réceptacle a un axe qui est généralement parallèle à la direction de la pointe.

4. Le dispositif chirurgical de la revendication 2 ou de la revendication 3 dans lequel le réceptacle a un trou de drainage externe (409).

5. Le dispositif chirurgical de la revendication 1 dans lequel l'élément allongé inclut un canal (540) dans au moins une portion de sa longueur, et l'attribut de mise en prise est configuré pour se mettre en prise et glisser dans le canal jusqu'à un emplacement souhaité sur l'élément allongé.

6. Le dispositif chirurgical de la revendication 5 comprenant en outre :
au moins un élément d'arrêt opérationnel pour fixer l'attribut de mise en prise à l'emplacement souhaité sur l'élément allongé.

7. Le dispositif chirurgical de la revendication 1 comprenant en outre :
une pluralité d'attributs de mise en prise disposés à des emplacements respectifs sur l'élément allongé, chaque attribut de mise en prise étant destiné à se mettre en prise avec l'attribut complémentaire de l'instrument de frappe.

8. Le dispositif chirurgical de la revendication 7 dans lequel au moins certains des attributs de mise en prise ont des axes parallèles.

9. Le dispositif chirurgical de la revendication 7 dans lequel au moins certains des attributs de mise en prise ont des axes non parallèles.

10. Le dispositif chirurgical de la revendication 7 dans lequel au moins certains des attributs de mise en prise sont configurés pour se mettre en prise avec différentes configurations de l'attribut complémentaire de l'instrument de frappe.

11. Un système pour une utilisation dans la réalisation d'une procédure de microfracture, comprenant :
un instrument de frappe (701) incluant un élément allongé ayant une extrémité proximale, une extrémité distale, et un trou (719) dans l'élément allongé près de son extrémité distale, une tige de frappe (715) configurée pour passer à travers le trou dans l'élément allongé, et une surface d'impact placée à une extrémité de la tige de frappe ; et
un dispositif chirurgical (700) selon n'importe quelle revendication précédente,
dans lequel l'instrument de frappe inclut en outre un attribut complémentaire placé à une autre extrémité de la tige de frappe,
dans lequel l'attribut de mise en prise du dispositif chirurgical est opérationnel pour se mettre en prise avec l'attribut complémentaire de l'instrument de frappe, et
dans lequel l'élément allongé de l'instrument de frappe est configuré pour être disposé de manière généralement parallèle à un axe longitudinal du dispositif chirurgical afin de permettre la mise en prise de l'attribut de mise en prise avec l'attribut complémentaire, moyennant quoi, au moment où l'on frappe la surface d'impact de l'instrument de frappe dans une direction généralement parallèle à une direction de la pointe du dispositif chirurgical, une force est produite qui est translatée via l'élément allongé du dispositif chirurgical à travers la pointe.

12. Le système de la revendication 11 dans lequel l'élément allongé de l'instrument de frappe est configuré pour être au moins temporairement fixé au dispositif chirurgical.

13. Le système de la revendication 11 dans lequel l'attribut de mise en prise du dispositif chirurgical est configuré sous la forme d'un raccord femelle, et l'attribut complémentaire de l'instrument de frappe est configuré sous la forme d'un raccord mâle.

14. Le système de la revendication 11 dans lequel l'attribut de mise en prise du dispositif chirurgical est configuré sous la forme d'un raccord mâle, et l'attribut complémentaire de l'instrument de frappe est configuré sous la forme d'un raccord femelle.
